# EUROPÄISCHE PATENTSCHRIFT

(11) **EP 2 461 891 B1**
(45) Veröffentlichungstag und Bekanntmachung des Hinweises auf die Patenterteilung: **13.06.2018**
(21) Anmeldenummer: 10747561.8
(22) Anmeldetag: 03.08.2010
(51) Int. Cl.: B01D 53/30, G01N 33/18, G01N 1/22, G01N 1/40

(54) **VERFAHREN ZUR KONTINUIERLICHEN BESTIMMUNG DES GEHALTS WENIGSTENS EINER CN-VERBINDUNG IN EINER WÄSSRIGEN LÖSUNG**
METHOD FOR CONTINUOUSLY DETERMINING THE CONCENTRATION OF AT LEAST ONE CN COMPOUND IN AN AQUEOUS SOLUTION
PROCÉDÉ PERMETTANT DE DÉTERMINER EN CONTINU LA TENEUR EN AU MOINS UN COMPOSÉ CN D'UNE SOLUTION AQUEUSE

(30) Priorität: 03.08.2009 AT 12272009
(43) Veröffentlichungstag der Anmeldung: 13.06.2012
(73) Patentinhaber: voestalpine Stahl GmbH, 4020 Linz (AT)
(72) Erfinder: PARZERMAIR, Franz, A-4840 Pilsbach (AT); MOSER, Leopold, A-4751 Dorf/Pram (AT); NADERER, Manfred, A-4282 Pierbach (AT)
(74) Vertreter: Jell, Friedrich
(86) Internationale Anmeldenummer: PCT/AT2010/000282
(87) Internationale Veröffentlichungsnummer: WO 2011/014898

(56) Entgegenhaltungen:
- EP-A2- 0 573 715
- WO-A1-94/01754
- DD-A3- 275 154
- DD-A5- 292 324
- DE-A1-102006 026 044
- JP-A- 2008 076 235

## Beschreibung

Die Erfindung betrifft ein Verfahren zur kontinuierlichen Bestimmung des Gehalts wenigstens einer CN-Verbindung in einer wässrigen Lösung, bei dem in die wässrige Lösung ein Trägergas, insbesondere Druckluft, eingebracht und das eingebrachte Trägergas zumindest teilweise einem Gasanalysator, insbesondere einem HCN Gasanalysator, zugeführt wird, dessen Analysedaten bei der Bestimmung des Gehalts der CN-Verbindung in der wässrigen Lösung berücksichtigt werden.

Zum Detektieren von Cyanidverbindungen (CN-Verbindungen) in wässrigen Lösungen ist es aus dem Stand der Technik bekannt (DE102006026044A1, DD292324A5), zunächst die CN-Verbindungen aus der Flüssig- in eine Gasphase auszutreiben und dann anhand eines Gassensors bzw. Gasanalysators über die erfassten ausgetriebenen CN-Verbindungen, insbesondere über HCN, auf den Gehalt der CN-Verbindungen in der wässrigen Lösung rückschließen zu können. Zum Austreiben der CN-Verbindungen schlagen die genannten Dokumente vor, die wässrige Lösung mit einem Trägergas zu beaufschlagen. In der DE102006026044A1 wird vorgeschlagen, die Menge an zu messendem Gas über Gaszutrittsbestimmungsmitteln zu erfassen, um damit eine präzise Messung zu erreichen. In der DD292324A5 wird vorgeschlagen, pH-Wert und Temperatur geeignet einzustellen, um eine zuverlässige Messung zu erhalten. Derartige Mittel sind jedoch konstruktiv aufwendig und führen zu einem vergleichsweise anfälligen Verfahren. Außerdem verlangen derartige Vorrichtungen einen vergleichsweise aufwendigen Reaktor mit einer Temperiervorrichtung, um so für ein kontrolliertes Austreiben der CN-Verbindungen und damit für reproduzierbare Messergebnisse sorgen zu können. Solche Reaktoren erlauben jedoch keine schnelle Reaktion auf toxische Lösungen, also wässrige Lösungen mit erhöhtem Gehalt an CN-Verbindungen, sodass diese Verfahren zwar zum Nachweis des Gehalts von CN-Verbindungen verwendet, nicht jedoch zur Steuerung einer schnellen Reaktionskette dienen können.

Außerdem ist es bekannt (DD 275 154 A3), in eine wässrige Lösung ein Membranmodul einzubringen, durch das ein gegenüber der wässrigen Lösung isoliertes Trägergas strömt. Durch das Membranmodul kann Blausäure eintreten, das vom Trägergas aufgenommen einer gemeinsamen Analyse unterworfen werden kann. Weiter wird vorgeschlagen, für eine Messwertkorrektur die Temperatur der wässrigen Lösung zu berücksichtigen. Ein Membranmodul ist jedoch vergleichsweise anfällig gegenüber Verschmutzungen, sodass ein standfestes Verfahren und eine standfeste Vorrichtung nicht sichergestellt werden können. Außerdem kann eine allgemeine Messung der Temperatur einer wässrigen Lösung ein genaues Verfahren nicht ermöglichen.

Es ist daher die Aufgabe der Erfindung ein Verfahren der eingangs geschilderten Art derart auszugestalten, dass nicht nur auf einfache Weise eine ausreichend genaue Bestimmung des Gehalts von CN-Verbindungen in einer wässrigen Lösung ermöglicht, sondern dass auch ein kontinuierliches Verfahren zur Verfügung gestellt werden kann. Außerdem soll das Verfahren reaktionsschnell sein.

Die Erfindung löst die gestellt Aufgabe durch die Merkmale des Anspruchs

Wird bei der Bestimmung des Gehalts an CN-Verbindungen die Temperatur der mit Trägergas beaufschlagten wässrigen Lösung berücksichtigt, dann kann eine treffende Temperaturmessung für eine Messkorrektur geschaffen, weil gerade die Temperatur des Teils der Lösung berücksichtigt wird, der mit dem Verfahren Bestimmung des Gehalts an CN-Verbindungen zusammenwirkt. Außerdem kann damit vorteilhaft ein reaktionsschnelles Verfahren ermöglicht werden, weil im Gegensatz zum Stand der Technik die wässrige Lösung vor dem Austreiben der CN-Verbindung nicht auf Temperatur gebracht werden muss, um so eine reproduzierbare und genaue Berechnung des Gehalts an CN-Verbindungen schaffen zu können. Unter Berücksichtigung der aktuellen und treffenden Temperatur der wässrigen Lösung, nämlich vom dem mit Trägergas beaufschlagten Teil der wässrigen Lösung, kann daher stets eine hohe Genauigkeit des Verfahrens gewährleistet werden, weil anhand dieser besonderen Temperaturerfassung die Parameter zum Rückschließen auf den Gehalt der CN-Verbindung in der wässrigen Lösung über die aktuellen Analysedaten des Gasanalysators anpassbar werden. Aufwendige Gaszutrittsmittel, Membranmodule und andere Maßnahmen zu Erhöhung der Messgenauigkeit können so vernachlässigt werden - weil ja das Trägergas einfach in die Lösung eingebracht wird - was nicht nur zu einem kostengünstigen sondern auch aufgrund seiner Einfachheit und Robustheit zu einem standfesten Verfahren führen kann. Die Erfindung zeichnet sich daher nicht nur durch seine Standfestigkeit, sondern auch durch die Möglichkeit der kontinuierlichen Bestimmung des CN-Gehalts von wässrigen Lösungen gegenüber dem Stand der Technik aus.

Einfache Verfahrensbedingungen in der Bestimmung des CN-Gehalts ergeben sich, wenn in Abhängigkeit der Temperatur ein veränderter Korrekturwert berücksichtigt wird. Dem Verfahren kann nämlich so in schneller Weise über die aktuelle Temperatur ein passender Korrekturwert zugeführt werden, sodass selbst mit einem geringen Rechenaufwand ein genaues Verfahren gewährleistet werden kann.

Das Austreiben der CN-Verbindung in eine Gasphase kann verbessert werden, in dem das Trägergas in die wässrige Lösung eingedüst wird.

Wird der wässrigen Lösung ein Stabilisator, beispielsweise CuSu, zugesetzt, dann kann mit der Behinderung des Übergangs in einen energieärmeren Zustand die Genauigkeit des Verfahrens verbessert werden.

Um den Einfluss von äußeren Störgrößen vermindern zu können, kann vorgesehen sein, dass die wässrige Lösung mit Trägergas in einem gasdicht abgeschlossenen Reaktor beaufschlagt wird. Damit kann nicht nur eine genaue Bestimmung des Gehalts an CN-Verbindungen gewährleistet werden, sondern es können damit auch die konstruktiven Vorraussetzungen für ein Zuführen des Trägergases zum Gassensor vereinfacht werden, was ein kostengünstiges Verfahren schaffen kann.

Wird die wässrige Lösung im pH Wert gesenkt, dann kann ein Austreiben der CN-Verbindung durch das Trägergas vorteilhaft verbessert werden. Solch eine Senkung kann beispielsweise vor dem oder gleichzeitig mit dem Einbringen des Trägergases in die wässrige Lösung erfolgen, in dem beispielsweise Salzsäure zugesetzt oder zur Bildung des Trägergases Kohlensäure verwendet wird.

Als besonders vorteilhaft kann sich hinsichtlich genauer und reproduzierbarer Verfahrensbedingungen herausstellen, wenn der pH Wert der wässrigen Lösung auf kleiner gleich vier eingestellt ist.

Zur Durchführung des Verfahrens wird eine Vorrichtung vorgeschlagen, bei der Reaktor einen die Temperatur von der wässrigen Lösung aufnehmenden Temperatursensor aufweist, der mit der Recheneinheit zur zusätzlichen Berücksichtigung der Temperatur der wässrigen Lösung bei der Bestimmung des Gehalts der CN-Verbindung verbunden ist.

Weist Reaktor einen die Temperatur von der wässrigen Lösung aufnehmenden Temperatursensor auf, der mit der Recheneinheit verbunden, dann kann es auf einfache Weise möglich werden, gerade diese Temperatur der wässrigen Lösung bei der Bestimmung des Gehalts der CN-Verbindung zu berücksichtigen, die entscheidend für die Genauigkeit des Verfahrens sein kann. Es hat sich nämlich herausgestellt, dass nicht die allgemeine Temperatur der wässrigen Lösung, sondern die Temperatur des Teils der wässrigen Lösung, die mit Trägergas beaufschlagt wird, eine besondere Bedeutung zur Messwertkorrektur zukommt. Es kann daher eine besonders genaue und standfeste Vorrichtung geschaffen werden. Außerdem kann auf aufwendige Gaszutrittsmittel, Membranmodule und andere Maßnahmen verzichtet werden, was zu einer einfach bedienbaren und kostengünstigen Vorrichtung führen kann.

Wird der Temperatursensor wenigstens im Nahebereich der mit Trägergas beaufschlagten wässrigen Lösung vorgesehen, dann können sich einfache Konstruktionsbedingungen zur genauen Messung ergeben. Ein aufwendiges Rückschließen auf die betreffende Temperatur bei einem Vorsehen des Sensors an einer anderen Stelle der wässrigen Lösung kann so vermieden werden.

In den Figuren ist beispielsweise der Erfindungsgegenstand anhand von Ausführungsbeispielen dargestellt. Es zeigen
- Fig. 1: eine schematische Ansicht auf ein erstes Ausführungsbeispiel einer erfindungsgemäßen Vorrichtung zur Durchführung des Verfahrens,
- Fig. 2: einen beispielsweise dargestellten Verlauf zu einem Korrekturwert in Abhängigkeit zur Temperatur der wässrigen Lösung und
- Fig. 3: eine vereinfachte zweite Ausführungsform der nach Fig. 1 dargestellten Vorrichtung.

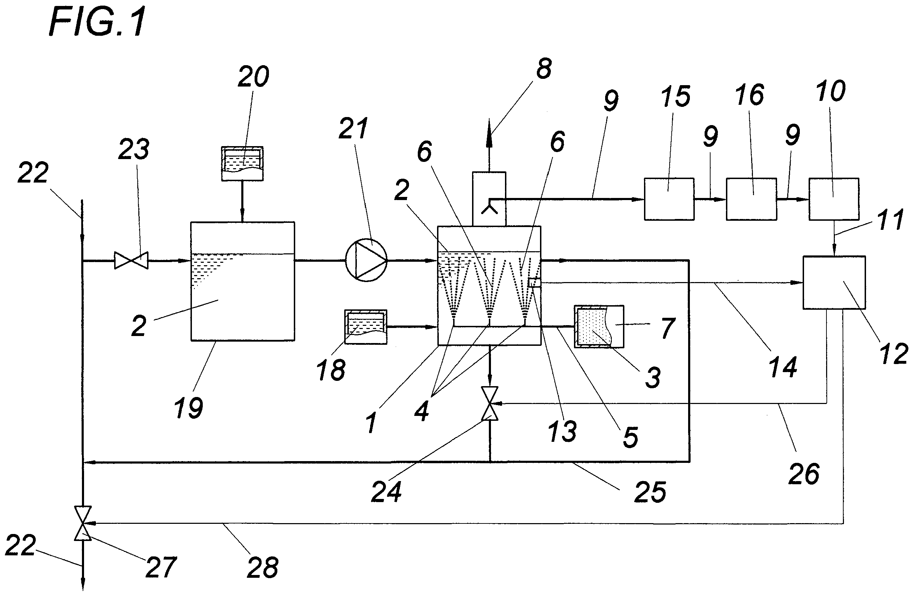

Die nach Fig. 1 beispielsweise dargestellte erfindungsgemäße Vorrichtung zur Durchführung des erfindungsgemäßen Verfahrens weist einen Reaktor 1 auf, in dem eine wässrige Lösung 2 vorgesehen ist, die nicht näher dargestellte Cyanidverbindungen (CN-Verbindungen) enthält. In diese wässrige Lösung 2 wird ein Trägergas 3 über Düsen 4, die an einer Trägergasleitung 5 vorgesehen sind, eingebracht, sodass sich kegelförmige Ausperlungen 6 ergeben können. Das Trägergas 3 ist in einem Trägergasbehälter 7 gelagert, der an die Trägergasleitung 5 über ein nicht näher dargestelltes Ventil angeschlossen ist. Die im Reaktor 1 entstehende Abluft 8, die zumindest teilweise das eingebrachte Trägergas 3 aufweist, wird über eine Gasprobenleitung 9 einem Gasanalysator 10 zugeführt, dessen Analysedaten 11 einer Recheneinheit 12 zur Berechnung des Gehaltes einer CN-Verbindungen und/oder mehrerer CN-Verbindungen in der wässrigen Lösung 2 zur Verfügung gestehen. Um eine vorteilhafte und insbesondere reaktionsschnelle Bestimmung schaffen, wird erfindungsgemäß vorgesehen, bei dieser Bestimmung die Temperatur der wässrigen Lösung 2 im Reaktor 1 berücksichtigt wird. Zu diesem Zweck ist im Behälter 2 ein Temperatursensor 13 vorgesehen, dessen Temperaturdaten 14 der Recheneinheit 12 zur Verfügung gestellt werden. Insbesondere ist dieser Temperatursensor 13 so vorgesehen, dass dieser auch die Temperatur der mit Trägergas beaufschlagten Lösung 2 im Reaktor 1 messen kann. Nach Fig. 1 befindet sich dieser Temperatursensor 13 im Reaktor 1; erfindungsgemäß ist er im Bereich der beaufschlagten wässrigen Lösung 2, um so exakte Temperaturdaten 14 von diesem Teil der wässrigen Lösung 2 aufnehmen zu können. Anhand der Temperaturdaten 14 kann so auf einfache Weise der Zusammenhang der kontinuierlichen Ergebnisse der Gasmessung auf der einen Seite und des aktuellen Gehalts der CN-Verbindung in der wässrigen Lösung 2 auf der anderen Seite angepasst bzw. verbessert werden, sodass selbst bei vergleichsweise starken Temperaturschwankungen in der zugeführten wässrigen Lösung 2 eine hohe Genauigkeit in der Bestimmung ermöglicht werden kann. Im Allgemeinen ist auch vorstellbar, für jede Düse 4 einen eigenen Temperatursensor 13 vorzusehen, damit die Temperatur der von der jeweiligen Düse beaufschlagten wässrigen Lösung 2 erfasst und damit die Genauigkeit noch weiter verbessert werden kann, was aber nicht dargestellt worden ist.

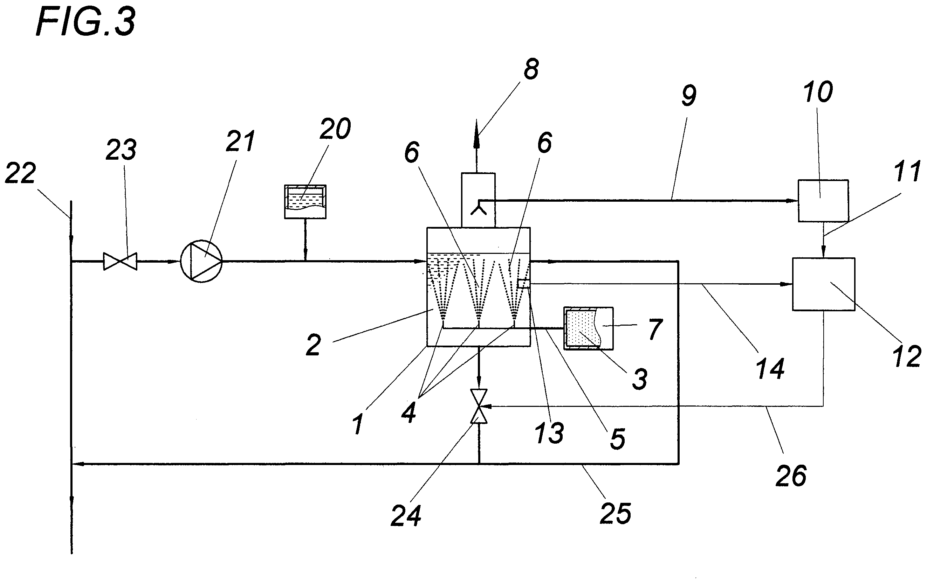

Insbesondere haben sich HCN- Gasanalysatoren 10 als besonders vorteilhaft herausgestellt, um die HCN Gase und/oder deren Konzentration im zugeführten Trägergas 3 zu messen, welche HCN Gase aus der wässrigen Lösung damit ausgetrieben werden. Zur Verbesserung der Gasmessung kann in der Gasprobenleitung 9 ein Gaskühler 15 und eventuell auch noch ein Selektivfilter 16 vorgesehen sein, der beispielsweise gegen H2S, SO2 und NO wirkt, um so Störeinflüsse auf den Gasanalysator 10 zu verringern. Eventuell kann auch noch eine nicht näher dargestellte Messgaspumpe vor dem Gasanalysator 10 vorgesehen sein. Gemäß Fig. 3 kann aber, wie im alternativen Ausführungsbeispiel dargestellt, darauf gänzlich verzichtet werden.

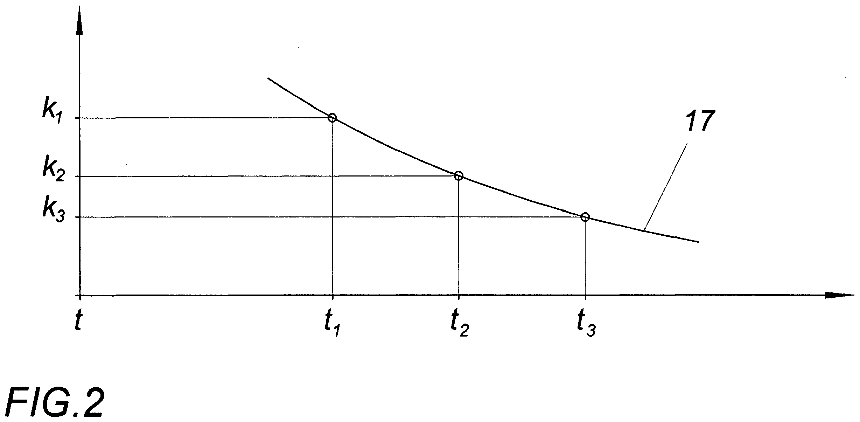

Eine einfache Berechnungsmöglichkeit ergibt sich, wenn in Abhängigkeit der jeweiligen Temperatur t1, t2 oder t3 der wässrigen Lösung 2 ein veränderter Korrekturwert k1, k2 oder k3 berücksichtigt wird, was der Fig. 2 besser entnommen werden kann. So ist bei einer Temperatur t1 der Korrekturwert k1 anhand der Korrekturwertkurve 17 einfach zu bestimmen bzw. abzulesen, welche Korrekturwertkurve 17 beispielsweise eine E-Kurve darstellen kann.

Zur Verbesserung der Genauigkeit des Verfahrens kann der wässrigen Lösung im Reaktor 2 ein Stabilisator 18, zugeführt werden (beispielsweise CuSu), wie in Fig. 1 dargestellt. Vorteilhaft ist der Reaktor 1 gasdicht ausgeführt, um so Störeinflüsse vermeiden zu können.

Vor dem Einbringen des Trägergases 3 wird die wässrige Lösung 2 im pH Wert gesenkt. Zu diesem Zweck wird Salzsäure 20 der wässrigen Lösung 2 zugesetzt, was in der Leitung nach der Pumpe 21 gemäß Fig. 3 oder in einem Vorbehandlungsbehälter 19 nach Fig. 1 durchgeführt werden kann.

Vorteilhafte Verfahrensbedingungen ergeben sich, wenn der pH Wert der wässrigen Lösung 2 auf vier oder dazu kleiner eingestellt bzw. diesbezüglich geregelt wird, bevor die wässrige Lösung 2 dem Reaktor 1 zugeführt wird. Der Einfachheit halber ist die Regelung nicht näher dargestellt. Zur Druckverbindung der Zuleitung 19 mit dem Reaktor 1 kann beispielsweise eine Förderpumpe 21 vorgesehen sein, über die auch ein dosiertes Einbringen der wässrigen Lösung 2 gesteuert werden kann.

Die zu analysierende wässrige Lösung 2 kann der Abwasserleitung 22 über ein Ventil 23 mit Hilfe der Pumpe 21 entnommen werden, wobei die wässrige Lösung 2 nach der Analyse vom Reaktor 1 über eine Abflussleitung 25 der Abwasserleitung 22 insbesondere drucklos zugegeben werden kann.

Um den Reaktor 1 von eventuellen Schwebstoffen reinigen zu können, ist es der Recheneinheit 12 möglich, über ein Ablassventil 24 den Reaktor 1 wenigstens teilweise abzulassen und so über die Abflussleitung 25 und weiter über die Abwasserleitung 22 zu entleeren.

Erfindungsgemäß kann sich durch das reaktionsschnelle Verfahren die Möglichkeit eröffnen, toxisches Abwasser bzw. wässrige Lösungen 2 mit CN-Verbindungen vor dem Verunreinigen von angeschlossenen Systemen zurückzuhalten, welche Systeme nicht näher dargestellt worden sind. Zu diesem Zweck kann beispielsweise in der Abwasserleitung 22 eine als Ventil 27 ausgebildete Einrichtung vorgesehen, die in Abhängigkeit der kontinuierlichen Bestimmung des Gehalts wenigstens einer CN-Verbindung in einer wässrigen Lösung 2 das Abwasser zurückhalten kann. Zu diesem Zweck ist eine Steuerleitung 28 vorgesehen, anhand der die Recheneinheit 12 das Ventil 27 betätigen kann.

## Patentansprüche

1. Verfahren zur kontinuierlichen Bestimmung des Gehalts wenigstens einer CN-Verbindung in einer wässrigen Lösung (2), bei dem in die wässrige Lösung (2) ein Trägergas (3), insbesondere Druckluft, eingebracht und das eingebrachte Trägergas (3) zumindest teilweise einem Gasanalysator (10), insbesondere einem HCN Gasanalysator, zugeführt wird, dessen Analysedaten (11) bei der Bestimmung des Gehalts der CN-Verbindung in der wässrigen Lösung (2) berücksichtigt werden, **dadurch gekennzeichnet, dass** die Temperatur (t1, t2 bzw. t3) von der mit Trägergas (3) beaufschlagten wässrigen Lösung (2) gemessen, wobei hierfür der Temperatursensor (13) im Bereich der mit Trägergas (3) beaufschlagten wässrigen Lösung (2) vorgesehen ist, und bei der Bestimmung des Gehalts der CN-Verbindung diese gemessene Temperatur (t1, t2 bzw. t3) für eine Messkorrektur berücksichtigt wird.

2. Verfahren nach Anspruch 1, **dadurch gekennzeichnet, dass** in Abhängigkeit der Temperatur (t1, t2 bzw. t3) bei der Bestimmung des Gehalts der CN-Verbindung ein veränderter Korrekturwert (k1, k2 bzw. k3) berücksichtigt wird.

3. Verfahren nach Anspruch 1 oder 2, **dadurch gekennzeichnet, dass** das Trägergas (3) eingedüst und damit in die wässrige Lösung (2) eingebracht wird.

4. Verfahren nach Anspruch 1, 2 oder 3, **dadurch gekennzeichnet, dass** das eingedüste Trägergas (3) in der wässrigen Lösung (2) kegelförmig ausperlt.

5. Verfahren nach einem der Ansprüche 1 bis 4, **dadurch gekennzeichnet, dass** dass der wässrigen Lösung (2) ein Stabilisator (18) zugesetzt wird.

6. Verfahren nach einem der Ansprüche 1 bis 5, **dadurch gekennzeichnet, dass** die wässrige Lösung (2) mit Trägergas (3) in einem gasdicht abgeschlossenen Reaktor (1) beaufschlagt wird.

7. Verfahren nach einem der Ansprüche 1 bis 6, **dadurch gekennzeichnet, dass** die wässrige Lösung (2) im pH Wert gesenkt wird, insbesondere unter Zugabe von Säure.

8. Verfahren nach Anspruch 7, **dadurch gekennzeichnet, dass** der pH Wert der wässrigen Lösung (2) auf kleiner gleich vier eingestellt wird.

9. Vorrichtung mit einem Gasanalysator (10), insbesondere einem HCN Gasanalysator, mit einem die wässrige Lösung (2) aufweisenden Reaktor (1), der eine in die wässrige Lösung (2) des Reaktors (1) einmündende Trägergasleitung (5) zum Einbringen von Trägergas (3) sowie eine über der wässrigen Lösung (2) des Reaktors (1) einmündende Gasprobenleitung (9) zum zumindest teilweisen Zuführen des eingebrachten Trägergas (3) zum Gasanalysator (10) zur Gasanalyse aufweist, und mit einer mit dem Gasanalysator (10) verbundenen Recheneinheit (12) zur kontinuierlichen Bestimmung des Gehalts wenigstens einer CN-Verbindung in der wässrigen Lösung (2) unter Berücksichtigung der Analysedaten des Gasanalysators (10), **dadurch gekennzeichnet, dass** der Reaktor (1) einen die Temperatur (t1, t2 bzw. t3) von der mit Trägergas (3) beaufschlagten wässrigen Lösung (2) aufnehmenden Temperatursensor (13) aufweist, der mit der Recheneinheit (12) zur zusätzlichen Berücksichtigung dieser gemessenen Temperatur (t1, t2 bzw. t3) der wässrigen Lösung (2) bei der Bestimmung des Gehalts der CN-Verbindung für eine Messkorrektur verbunden ist, wobei der Temperatursensor (13) im Bereich der mit Trägergas (3) beaufschlagten wässrigen Lösung (2) vorgesehen ist.

10. Vorrichtung nach Anspruch 9, **dadurch gekennzeichnet, dass** die Trägergasleitung (5) über eine Düse (4) in die wässrige Lösung (2) einmündet.

11. Vorrichtung nach Anspruch 10, **dadurch gekennzeichnet, dass** das eingedüste Trägergas (3) in der wässrigen Lösung (2) eine kegelförmige Ausperlung (6) ausbildet.

## Claims

1. A method for continuously determining the concentration of at least one CN compound in an aqueous solution (2) comprising:
introducing a carrier gas (3), more particularly compressed air, into an aqueous solution (2); and conveying at least part of the introduced carrier gas (3) to a gas analyzer, more particularly a HCN gas analyzer, whose analysis data (11) are taken into account in determining a concentration of the CN compound in the aqueous solution (2), **characterized in that**, the temperature (t1, t2 or t3) of the aqueous solution (2) being applied with the carrier gas (3) is measured, wherein for this purose the temperature sensor (13) is located in an aerea of the aqueous solution (2) being applied with the carrier gas (3), and this determined temperature (t1, t2 or t3) is taken into account for a measurement correction in determining the concentration of the CN compound.

2. The method according to claim 1, **characterized in that**, an altered correction value (k1, k2 or k3) dependent on the temperature (t1, t2 or t3) is taken into account when determining the concentration of the CN compound.

3. The method according to claim 1 or 2, **characterized in that**, the carrier gas (3) is injected into and therefor introduced into the aqueous solution (2).

4. The method according to claim 1, 2 or 3, **characterized in that**, the injected carrier gas (3) bubbles out in an conical manner in the aqueous solution (2).

5. The method according to any of the claims 1 to 4, **characterized in that**, a stabilizer (18) is added to the aqueous solution (2).

6. The method according to any of the claims 1 to 5, **characterized in that**, aqueous solution (2) is acted on with carrier gas (3) in an reactor (1) that is sealed in a gastight fashion.

7. The method according to any of the claims 1 to 6, **characterized in that**, the pH value of the aqueous solutio (2) is lowered, more particularly by adding acid.

8. The method according to claim 7, **characterized in that**, the pH value of the aqueous solutio (2) is set to equal or less than four.

9. A device with a gas analyzer (10), more particularly a HCN gas analyzer, comprising:
a reactor (1) that contains the aqueous solution (2) and has a carrier gas line (5) that feeds into the aqueous solution (2) of the reactor (1) in order to introduce carrier gas (3) and has a gas sampling line (9), which is connected at a location above the aqueous solution (2) of the reactor (1) for at least partially adding the introduced carrier gas (3) to the gas analyzer (1) for gas analysis;
a computing unit (12) connected to the gas analyzer (1) for continuously determining a concentration of at least one CN compound in the aqueous solution (2) while taking into account analysis data of the gas analyzer (10), **characterized in that**, the reactor (1) comprises a temperature sensor (13) recording the temperature (t1, t2 or t3) of the of the aqueous solution (2) being applied with the carrier gas (3), which is connected with the computing unit (12) for additionally taking into account this determined temperature (t1, t2 or t3) of the aqueous solution (2) for determining the concentration of the CN compound for a measurement correction, wherein the temperature sensor (13) is located in the aerea of the aqueous solution (2) being applied with the carrier gas (3).

10. The device according to claim 9, **characterized in that**, the carrier gas line (5) feeds into the aqueous solution (2) via a nozzle (4).

11. The device according to claim 10, **characterized in that**, the injected carrier gas (3) forms a conical bubbling (6) in the aqueous solution (2).

## Revendications

1. Procédé de détermination en continu de la teneur en au moins un composé CN d'une solution aqueuse (2), dans lequel un gaz porteur (3), en particulier de l'air comprimé, est introduit dans la solution aqueuse (2), et le gaz porteur introduit (3) est amené au moins en partie à un analyseur de gaz (10), en particulier un analyseur de gaz HCN, dont les données d'analyse (11) sont prises en compte dans la détermination de la teneur en composé CN de la solution aqueuse (2), **caractérisé en ce que** la température (t1, t2 ou t3) de la solution aqueuse (2) exposée au gaz porteur (3) est mesurée, le capteur de température (13) étant prévu à cet effet dans la zone de la solution aqueuse (2) exposée au gaz porteur (3), et que cette température mesurée (t1, t2 ou t3) est prise en compte pour une correction de mesure lors de la détermination de la teneur en composé CN.

2. Procédé selon la revendication 1, **caractérisé en ce qu'**en fonction de la température (t1, t2 ou t3), une valeur de correction modifiée (k1, k2 ou k3) est prise en compte lors de la détermination de la teneur en composé CN.

3. Procédé selon la revendication 1 ou 2, **caractérisé en ce que** le gaz porteur (3) est injecté et ainsi introduit dans la solution aqueuse (2).

4. Procédé selon la revendication 1, 2 ou 3, **caractérisé en ce que** le gaz porteur injecté (3) forme une effervescence en cône dans la solution aqueuse (2).

5. Procédé selon l'une des revendications 1 à 4, **caractérisé en ce qu'**un stabilisateur (18) est ajouté à la solution aqueuse (2).

6. Procédé selon l'une des revendications 1 à 5, **caractérisé en ce que** la solution aqueuse (2) est exposée au gaz porteur (3) dans un réacteur (1) fermé et étanche aux gaz.

7. Procédé selon l'une des revendications 1 à 6, **caractérisé en ce que** le pH de la solution aqueuse (2) est abaissé, en particulier par addition d'acide.

8. Procédé selon la revendication 7, **caractérisé en ce que** le pH de la solution aqueuse (2) est réglé à une valeur inférieure ou égale à quatre.

9. Dispositif comprenant un analyseur de gaz (10), en particulier un analyseur de gaz HCN, un réacteur (1) contenant la solution aqueuse (2), qui présente une conduite de gaz porteur (5) débouchant dans la solution aqueuse (2) du réacteur (1) pour l'introduction d'un gaz porteur (3) et une conduite de prélèvement de gaz (9) débouchant au-dessus de la solution aqueuse (2) du réacteur (1) pour l'amenée au moins partielle du gaz porteur introduit (3) à l'analyseur de gaz (10) pour l'analyse du gaz, et comprenant une unité de calcul (12) reliée à l'analyseur de gaz (10) pour la détermination en continu de la teneur en au moins un composé CN de la solution aqueuse (2) en tenant compte des données d'analyse de l'analyseur de gaz (10), **caractérisé en ce que** le réacteur (1) présente un capteur de température (13) détectant la température (t1, t2 ou t3) de la solution aqueuse (2) exposée au gaz porteur (3), qui est relié à l'unité de calcul (12) pour la prise en compte supplémentaire de cette température mesurée (t1, t2 ou t3) de la solution aqueuse (2) pour une correction de la mesure lors de la détermination de la teneur en composé CN, le capteur de température (13) étant prévu dans la zone de la solution aqueuse (2) exposée au gaz porteur (3).

10. Dispositif selon la revendication 9, **caractérisé en ce que** la conduite de gaz porteur (5) débouche dans la solution aqueuse (2) par l'intermédiaire d'un injecteur (4).

11. Appareil selon la revendication 10, **caractérisé en ce que** le gaz porteur (3) injecté forme une effervescence (6) en cône dans la solution aqueuse (2).
